# DEMANDE DE BREVET EUROPEEN

(11) **EP 3 097 928 A1**
(43) Date de publication de la demande: **30.11.2016**
(21) Numéro de dépôt: 15305806.0
(22) Date de dépôt: 28.05.2015
(51) Int. Cl.: A61K 48/00, A61K 31/737, A61P 25/00, A61P 9/10

(54) **COMPOSITION POUR LE TRAITEMENT DES LESIONS CEREBRALES**

(71) Demandeur: Organes Tissus Régénération Réparation Remplacement, 75001 Paris (FR); Barritault, Denis, 75001 Paris (FR); CENTRE NATIONAL DE LA RECHERCHE SCIENTIFIQUE -CNRS-, 75794 Paris Cedex 16 (FR); Université de Caen Basse Normandie, 14032 Caen Cedex (FR)
(72) Inventeur: BARRITAULT, Denis, 75001 PARIS (FR); BERNAUDIN, Myriam, 14990 BERNIERES SUR MER (FR); TOUZANI, Omar, 14310 VILLY-BOCAGE (FR); TOUTAIN, Jérôme, 14260 ONDEFONTAINE (FR); QUITTET, Marie-Sophie, 76600 LE HAVRE (FR)
(74) Mandataire: Novagraaf Technologies

(57) **Abrégé**

La présente invention se rapporte à une composition pharmaceutique comprenant un polymère biocompatible de formule générale (I) et une cellule eucaryote pour son utilisation comme médicament pour la prévention et/ou le traitement de lésions tissulaires du système nerveux central dues à une ischémie vasculaire cérébrale. La présente invention se rapporte également à un kit pharmaceutique comprenant un polymère biocompatible de formule générale (I) et une cellule eucaryote pour la prévention et/ou le traitement de lésions tissulaires du système nerveux central dues à une ischémie vasculaire cérébrale.

La présente invention trouve une application notamment dans les domaines pharmaceutiques, humaine, et vétérinaire.

## Description

### Domaine technique

La présente invention se rapporte à une composition pharmaceutique pour son application comme médicament pour la prévention et/ou le traitement de lésions tissulaires du système nerveux central dues à une ischémie vasculaire cérébrale.

La présente invention se rapporte également à un kit pharmaceutique pour la prévention et/ou le traitement de lésions tissulaires du système nerveux central dues à une ischémie vasculaire cérébrale.

La présente invention trouve une application notamment dans les domaines pharmaceutiques, humaine, et vétérinaire.

Dans la description ci-dessous, les références entre parenthèses ( ) renvoient à la liste des références présentée à la fin du texte.

### Etat de la technique

Les accidents vasculaires cérébraux (AVC) représentent toujours la première cause de morbidité et la troisième cause de mortalité chez l'Homme dans les pays industrialisés. Le tribut à payer à cette pathologie reste très lourd : 10 à 12 % de l'ensemble des décès après 65 ans ainsi que des séquelles physiques, cognitives ou psychologiques chez plus de la moitié des victimes. Selon l'OMS, 15 millions de personnes en souffrent à travers le monde chaque année. Parmi ceux-ci, 5 millions meurent et 5 autres millions sont handicapés à vie. En Europe, on estime le nombre de décès par AVC à environ 650 000 chaque année. En conséquence, le retentissement socio-économique des AVC est très important (5.3 milliards d'Euro en 2007 en France (Chevreul et al., 2013).

L' AVC est défini comme la diminution de l'apport sanguin dans une zone cérébrale donnée. Il existe deux types d'AVC: l'accident hémorragique, qui correspond à la fuite du sang du compartiment vasculaire vers le compartiment cellulaire résultant de la rupture du vaisseau sanguin, et le type ischémique dont sont victimes 80% des patients atteints d'AVC. Ce dernier est dû à la diminution du flux sanguin causée par un embole correspondant à un caillot qui se détacherait de la périphérie et serait entraîné jusqu'à l'artère cérébrale, ou par une plaque d'athérosclérose finissant par occlure totalement la lumière du vaisseau. L'artère la plus fréquemment concernée par cette occlusion est l'artère Sylvienne ou artère cérébrale moyenne (MCA, Middle Cerebral Artery). Il s'agit d'une artère irrigant une majeure partie de l'hémisphère cérébrale et dont l'occlusion engendre un handicape sensori-moteur ou cognitif important (hémiplégie, hémiparésie, agnosie, déficits mnésiques...) (Cramer, 2008; Jaillard et al., 2009).

### Traitement de l'AVC ischémique

L'ischémie cérébrale peut être définie comme une inadéquation entre l'apport sanguin et la demande métabolique. Ceci a pour origine une diminution du débit sanguin cérébral qui peut être transitoire ou durable. La lésion cérébrale qui accompagne une ischémie focale consiste généralement en un foyer sévèrement atteint et une zone périphérique dont la viabilité est précaire ; cette zone, dénommée pénombre, peut être recrutée par le processus de nécrose sauf si une intervention thérapeutique est instituée à temps (Touzani et coll., 2001). La pénombre ischémique représente donc la cible de toute intervention thérapeutique pendant la phase aigue de l'ischémie cérébrale

Malgré l'important problème de santé publique que représente l'AVC ischémique, l'arsenal thérapeutique pour lutter contre ce dernier est faible. Actuellement, seule la thrombolyse par le t-PA (activateur tissulaire du plasminogène) est approuvée par les autorités de santé. Cependant, l'utilisation du t-PA est restreinte de par sa faible fenêtre thérapeutique à savoir de 3 à 4,5h après la survenue de l'AVC et les nombreuses contre-indications qui lui sont associées liées aux risques d'hémorragie cérébrale (absence de traitement fluidifiant sanguin, absence d'accident ischémique (cérébral ou cardiaque) dans les 3 précédents mois, absence d'hémorragie gastro-intestinale ou urinaire dans les 21 derniers jours, absence de saignement, pression artérielle < à 185/110 mmHg systolique/diastolique...). D'après Lees et collaborateurs (2010), l'administration du rt-PA après un délai supérieur à 4h30 induit un risque d'hémorragie cérébrale significativement plus important que chez des patients non traités, et est associée à une balance bénéfice-risque non favorable (Lees et al., 2010). Ainsi, on estime que seuls 3 à 5 % des patients peuvent avoir recours à ce traitement (Adeoye et al., 2011) et malgré la sélection stricte des patients, on évalue que 13% d'entre eux développeront une hémorragie cérébrale à la suite de l'administration du rt-PA.

Il existe donc un réel besoin de trouver une nouvelle composition/médicament palliant ces défauts, inconvénients et obstacles, en particulier d'une composition permettant notamment de traiter/stopper un AVC présentant notamment une fenêtre de traitement élargie et/ou diminuant/ éliminant les effets secondaires dus au traitement.

A part la thrombolyse par le t-PA, de nombreuses investigations chez l'animal ont montré une efficacité éventuelle de plusieurs stratégies thérapeutiques visant à protéger les neurones contre l'ischémie (Kaur et al., 2013). Parmi ces stratégies, on peut citer le blocage des canaux calciques, l'inhibition du stress oxydatif, la stimulation des récepteurs GABA A, l'inhibition des récepteurs NMDA et AMPA. Cependant, en clinique humaine, le succès de ces interventions thérapeutiques n'a pas été retrouvé (Kaur et al., 2013).

Il existe donc un réel besoin de trouver une nouvelle composition/médicament permettant de traiter l'AVC et ses conséquences tissulaires.

Etant donné les échecs dramatiques de plusieurs essais cliniques ayant testés des interventions thérapeutiques de neuroprotection après un AVC chez l'Homme, de nombreux auteurs s'orientent vers le développement de stratégies de réparation cérébrale applicables pendant la phase subaigüe ou chronique de la pathologie. Ces stratégies consistent en l'apport de facteurs neurotrophiques ou en la transplantation de cellules souches afin de favoriser la récupération fonctionnelle.

### Cellules souches et ischémie cérébrale

Plusieurs types de cellules souches ont été testés chez l'animal soumis à une ischémie cérébrale. Parmi les quelles on peut citer les cellules souches embryonnaires (ESC), les cellules souches pluripotentes induites (iPSC), les cellules souches neurales (CSN) et les cellules souches mésenchymateuses (CSM) (pour revue, voir Hao et al., 2014). Bien que les ESC et les iPSC aient montrées des effets bénéfiques chez l'animal après ischémie, leurs problèmes de disponibilités (pour les ESC) et leur capacité de se transformer en tumeurs limitent, pour l'instant, leur utilisation chez l'être humain. En effet, il a été démontré que ces cellules sont susceptibles d'être à l'origine de génération de tumeurs après injection.

Les cellules souches neurales (CSNs) sont retrouvées dans le tissu foetal, le tissu néonatal, chez le jeune mais également chez l'adulte. Les niches de cellules souches neuroblastiques chez l'Homme et chez l'animal sont la zone sous-ventriculaire (SVZ) et la zone sous-granulaire du gyrus denté (Seri et al., 2006). Ces cellules, bien que déjà orientées dans leur différenciation sont dites souches car elles sont capables, dans le cadre de protocole de différenciation particuliers, de se différencier en neurones hippocampiques, en neurones corticaux ou encore en motoneurones ou interneurones. Il existe de nombreuses études dans la littérature qui ont montré des effets bénéfiques de la transplantation des CSNs après une ischémie cérébrale, par exemple tel que décrit dans le document Hao et collaborateurs, 2014. Par exemple, l'administration des CSNs dans la lésion corticale ischémique ou dans sa périphérie favorise la production de neuroblastes dans la SVZ. Une stimulation de l'arborisation dendritique ainsi que de la croissance axonale corrélées avec une augmentation de la récupération fonctionnelle est observée après leur administration chez le rat (Andres et al., 2011). Cependant, plusieurs contraintes limitent l'utilisation de ces cellules en clinique. En effet, l'isolement de ces cellules à partir de foetus est rendue difficile par les contraintes éthiques. Une autre source de CSNs serait la biopsie cérébrale de la SVZ qui ne peut être effectuée dans le cas de l'ischémie que post-mortem limitant grandement la quantité de ressources et rendant impossible le recours à l'autogreffe chez le patient.

Une autre piste explorée est l'utilisation d'autres cellules souches plus accessibles telles que les cellules souches mésenchymateuses (MSCs). Ces cellules ont été identifiées pour la première fois par Friedenstein et collaborateurs en 1970 qui ont caractérisé ces cellules comme adhérentes au plastique et rares (Friedenstein et al., 1970). Plusieurs sources ont été identifiées et utilisées dont majoritairement la moelle osseuse, mais également la pulpe dentaire (Yalvac et al., 2009; Yamagata et al., 2013), le follicule pileux (Wang et al., 2013), le placenta (In 't Anker et al., 2004) ou le cordon ombilical (Erices et al., 2000; Kranz et al., 2010)

Comme toutes les cellules souches, les MSCs peuvent se différencier en cellules spécialisées et s'auto-renouveler. Les MSCs sont capables de se différencier, in vitro en plusieurs types cellulaires et dans un environnement et des conditions adaptées, elles sont capables de se différencier vers un phénotype non mésenchymateuse comme le phénotype neuronale ou cardiomyocytaire (Esneault et al., 2008; Toma et al., 2002). La facilité d'accès et d'extraction de ces cellules à partir de la moelle osseuse et leur multiplication aisée et rapide pourrait permettre de réaliser des greffes autologues susceptibles de limiter l'utilisation de traitements immunosuppresseurs difficilement tolérables par les patients. Par ailleurs, les cellules souches mésenchymateuses n'expriment pas le complexe majeur d'histocompatibilité (CMH) de type II (HLA-DR ou HLA type II) et peu le CMH de type I (HLA-ABC ou HLA type I) au niveau membranaire. En outre, Di Nicola et collaborateurs en 2002 ont mis en évidence la diminution de la prolifération des lymphocytes T dans des conditions de co-culture avec des MSCs et ceux de façon dose dépendante et réversible (Di Nicola, 2002). Outre les lymphocytes T, les MSCs peuvent présenter une action anti-inflammatoire sur d'autres cellules de l'inflammation telles que les Natural Killer, les cellules dendritiques ou les macrophages (Aggarwal & Pittenger, 2005; Eckert et al., 2013). Les essais cliniques menés dans le cadre de pathologies cardiaques, nerveuses ou encore immunes n'ont a priori pas mis en évidence d'effets indésirables graves à la suite d'une administration de MSCs (Malgieri et al., 2010).

Dans l'ischémie cérébrale, un bénéfice fonctionnel post-ischémique obtenu après l'administration de MSCs a été mis en évidence par des études précliniques et quelques études cliniques comme résumées dans Hao et al., 2014, et Kaladka et Muir, 2014.

En ce qui concerne les études cliniques Bang et collaborateurs (2005) ont administré pour la première fois des MSCs à des patients ayant subi une ischémie cérébrale. Cette première étude a été réalisée sur peu de patients (5 vs 25 contrôles) mais a mis en évidence une absence de développement tumoral et une faisabilité éventuelle de l'administration autologue de MSCs chez des patients ayant subi un AVC. Une amélioration de la récupération fonctionnelle post-ischémique des patients traités a été observée par les auteurs 3 à 6 mois post-traitement. Ces résultats ont été confirmés en 2010 via une administration IV de MSCs chez 16 patients souffrant d'AVC. En particulier, une diminution relative de la mortalité des patients traités a été observée. Un effet bénéfique du traitement sur la récupération fonctionnelle a été montré sur une période d'observation de 5 ans à l'aide du mRS (Lee et al., 2010). Depuis, d'autres études ont permis de renforcer la notion de faisabilité et de sécurité de cette nouvelle stratégie thérapeutique (Bhasin et al., 2011; Suàrez-Monteagudo et al., 2009). Plusieurs phénomènes expliquent l'efficacité des MSCs tels que leur propriété paracrine concernant des facteurs de croissance neurogénique ou angiogénique (FGF2, le NGFb, l'EGF, le VEGF-A l'IGF1, BDNF).

Cependant, malgré les nombreux avantages qu'elles présentent, les MSCs ont une survie très limitée après une administration dans une zone ischémique. En effet, 99% des cellules meurent pendant les 24 premières heures et d'après Toma et collaborateurs (2002), seuls 0,5% des MSCs implantées dans un environnement ischémique survivent 4 jours après l'implantation. Plusieurs phénomènes expliquent cette perte cellulaire (Toma et al., 2002). En effet, l'inflammation, l'hypoxie, l'anoïkis (absence de support) ou les facteurs pro-apoptotiques présents dans le milieu environnants induisent le déclenchement de l'apoptose. De plus, l'ischémie cérébrale étant caractérisée par une réduction du débit sanguin cérébral, les cellules greffées sont donc en manque de substrats énergétiques indispensables à leur survie. Les neutrophiles et les macrophages recrutés dans la zone ischémiée vont, en outre, produire des radicaux oxygénés pour lesquels, Song et collaborateurs (Song, Cha, et al., 2010) ont mis en évidence, dans le cas de l'ischémie cardiaque, l'effet délétère sur l'attachement des cellules souches mésenchymateuses. Or, l'adhésion des cellules à la matrice extra-cellulaire du milieu environnant via les protéines d'intégrines induit un signal positif au sein de la cellule et une répression de l'apoptose, alors que le phénomène inverse se produit en cas de manque de support (Song, Song, et al., 2010). De plus, suite à une ischémie, la matrice extra-cellulaire est détruite par les métalloprotéases et la rémanence de ces dernières limite la reconstruction de la MEC. D'après Toma et collaborateurs, l'un des facteurs majeurs de mort des cellules injectées serait l'absence de support de pousse, induisant l'anoikis. Ce phénomène juxtaposé avec la présence des radicaux libres qui restreignent encore l'incorporation du greffon dans le tissu hôte. En outre, la régénération tissulaire dans le cas de l'ischémie est fortement dépendante de la vascularisation de la zone de cicatrisation.

Il existe également un réel besoin de trouver une nouvelle composition palliant les défauts, inconvénients et obstacles de l'art antérieur, en particulier d'une composition permettant notamment de traiter/stopper un AVC, de traiter les conséquences/effets d'un AVC, réduire les coûts et d'améliorer le schéma thérapeutique/posologique du traitement des AVC.

### Description de l'invention

La présente invention a précisément pour but de répondre à ces besoins en fournissant une composition pharmaceutique pour son application comme médicament pour la prévention et/ou le traitement de lésions tissulaires du système nerveux central dues à une pathologie hypoxique cérébrale, ladite composition comprenant
- un polymère biocompatible de formule générale (I) suivante

   AaXxYy (I)

   dans laquelle :
   A représente un monomère,
   X représente un groupement -R₁COOR₂ ou -R₉(C=O)R₁₀,
   Y représente un groupement O- ou N-sulfonate et répondant à l'une
   des formules suivante -R₃OSO₃R₄, -R₅NSO₃R₆, -R₇SO₃R₈ dans lesquelles :
      R₁, R₃, R₅ et R₉ représentent indépendamment une chaîne hydrocarbonée aliphatique, éventuellement ramifiée et/ou insaturée et qui contient éventuellement un ou plusieurs cycles aromatiques, R₂, R₄, R₆ et R₈ représentent indépendamment un atome d'hydrogène ou un cation,
      R₇ et R₁₀ représente indépendamment une liaison, une chaîne hydrocarbonée aliphatique, éventuellement ramifiée et/ou insaturée, « a » représente le nombre de monomères,
      « x » représente le taux de substitution des monomères A par des groupements X,
      « y » représente le taux de substitution des monomères A par des groupements Y, et
- une cellule eucaryote.

Dans la présente par « lésions tissulaires du système nerveux central » on entend toutes lésions tissulaires susceptibles d'apparaitre au niveau du système nerveux central. Il peut s'agir par exemple d'une lésion tissulaire due à un choc physique, par exemple lié à un traumatisme, une lésion tissulaire due à un choc ischémique, par exemple due à une diminution du débit sanguin cérébral transitoire et/ou durable lié par exemple à une occlusion vasculaire, une hémorragie vasculaire ou encore à un choc hypoxique

Dans la présente par « pathologie hypoxique cérébrale » on entend toute pathologie et/ou évènement susceptible de provoquer une diminution de l'apport d'oxygène au cerveau.

Il peut s'agir par exemple d'un accident vasculaire, un arrêt cardiaque, une hypotension, une ou plusieurs complications liées à l'anesthésie pendant l'intervention, une suffocation, un empoisonnement au monoxyde de carbone, une noyade, une inhalation de monoxyde de carbone ou de fumée, des lésions cérébrales, une strangulation, une crise d'asthme, un traumatisme, une lésion tissulaire due à un choc ischémique , une hypoxie périnatale...

Dans la présente par monomère on entend par exemple un monomère choisi dans le groupe comprenant les sucres, les esters, les alcools, les acides aminés ou les nucléotides.

Dans la présente, les monomères A constituent les éléments de base des polymères de formule I peuvent être identiques ou différents.

Dans la présente, l'association de monomères peut permettre de former un squelette polymérique, par exemple un squelette polymérique de nature polyester, polyalcool, polysaccharidique, du type des acides nucléiques ou des protéines.

Dans la présente parmi les polyesters, il peut s'agir par exemple de copolymères de biosynthèse ou synthèse chimique, par exemple des polyesters aliphatiques ou d'origine naturelle par exemple les polyhydroxyalconaotes.

Dans la présente, les polysaccharides et leurs dérivés peuvent être d'origine bactérienne, animale, fongique et/ou d'origine végétale. Il peut s'agir par exemple de polysaccharides à chaîne simple, par exemple les polyglucoses, par exemple le dextran, la cellulose, le bêta glucan, ou d'autres monomères comprenant des unités plus complexes, par exemple les xanthanes, par exemple le glucose, mannose et acide glucuronique ou encore des glucuronanes et glucoglucuronane.

Dans la présente, les polysaccharides d'origine végétale peuvent être à simple chaîne, par exemple la cellulose (glucose), les pectines (acide galacturonique), les fucanes, l'amidon ou plus complexe comme les alginates (acide galuronique et mannuronique).

Dans la présente, les polysaccharides d'origine fungique peuvent être par exemple le stéroglucane.

Dans la présente, les polysaccharides d'origine animale peuvent être par exemple les chitines ou le chitosan (glucosamine).

Le nombre de monomères A défini dans la formule (I) par « a » peut être tel que la masse desdits polymères de formule (I) est supérieure à environ 2000 daltons (ce qui correspond à 10 monomères de glucose). Le nombre de monomères A défini dans la formule (I) par « a » peut être tel que la masse desdits polymères de formule (I) est inférieure à environ 2000000 daltons (ce qui correspond à 10000 monomères de glucose). De façon avantageuse, la masse desdits polymères de formule (I) peut être comprise de 2 à 100 kdaltons.

Dans la présente, dans le groupement -R₁COOR₂ représentant X, R₁ peut être un alkyle en C₁ à C₆, par exemple un méthyl, un éthyl, un butyl, un propyl, un pentyl, de préférence un groupement méthyl, et R₂ peut être une liaison, un alkyle en C₁ à C₆, par exemple un méthyl, un éthyl, un butyl, un propyl, un pentyl, un groupement R₂₁R₂₂ dans lequel R₂₁ est un anion et R₂₂ un cation choisi dans le groupe des métaux alcalins.

De préférence, le groupement X est le groupement de formule -R₁COOR₂ dans laquelle R₁ est un groupement méthyle -CH₂- et R₂ un groupement R₂₁R₂₂ dans lequel R₂₁ est un anion et R₂₂ un cation choisi dans le groupe des métaux alcalins, de préférence le groupement X est un groupement de formule -CH₂-COO⁻.

Dans la présente, dans le groupement -R₉(C=O)R₁₀ représentant X, R₉ peut être un alkyle en C₁ à C₆, par exemple un méthyl, un éthyl, un butyl, un propyl, un pentyl, de préférence un groupement méthyl, et R₁₀ peut être une liaison, un alkyle en C₁ à C₆, par exemple un méthyl, un éthyl, un butyl, un propyl, un pentyl, un hexyl.

Dans la présente, dans le groupement répondant à l'une des formules suivantes -R₃OSO₃R₄, -R₅NSO₃R₆, -R₇SO₃R₈ et représentant le groupement Y, R₃ peut être une liaison, un alkyle en C1 à C6, par exemple un méthyl, un éthyl, un butyl, un propyl, un pentyl, de préférence un groupement méthyl, R₅ peut être une liaison, un alkyle en C₁ à C₆, par exemple un méthyl, un éthyl, un butyl, un propyl, un pentyl, de préférence un groupement méthyl, R₇ peut être une liaison, un alkyle en C₁ à C₆, par exemple un méthyl, un éthyl, un butyl, un propyl, un pentyl, de préférence un groupement méthyl, R₄, R₆ et R₈ peuvent être indépendamment un atome d'hydrogène ou un cation M⁺, par exemple M⁺ peut être un métal alcalin.

De préférence, le groupement Y est le groupement de formule R₇SO₃R₈ dans lequel R₇ est une liaison et R₈ est un métal alcalin choisi dans le groupe comprenant le sodium, le potassium, le rubidium et le césium, De préférence, le groupement Y est un groupement -SO₃⁻Na⁺

Le taux de substitution de l'ensemble des monomères A par les groupements Y défini dans la formule générale (I) par « y » peut être compris de 30 à 150%, et de préférence de l'ordre de 100%.

Dans la présente, la définition des taux de substitutions ci- dessus, on entend par un taux de substitution « x » de 100%, le fait que chaque monomère A du polymère de l'invention contient statistiquement un groupement X. De même, on entend par un taux de substitution « y » de 100%, le fait que chaque monomère du polymère de l'invention contient statistiquement un groupement Y. Les taux de substitution supérieurs à 100% traduisent le fait que chaque monomère porte statistiquement plus d'un groupement du type considéré ; à l'inverse, les taux de substitution inférieurs à 100% traduisent le fait que chaque monomère porte statistiquement moins d'un groupement du type considéré.

Les polymères peuvent également comprendre des groupements chimiques fonctionnels, désignés Z, différents de X et Y.

Dans la présente, les groupements Z peuvent être identiques ou différent, et peuvent indépendamment être choisis dans le groupe comprenant des acides aminés, des acides gras, des alcools gras, des céramides, ou des dérivés de ceux-ci, ou des séquences nucléotidiques d'adressages.

Les groupements Z peuvent également représenter des agents actifs identiques ou différents. Il peut s'agir par exemple d'agents thérapeutiques, d'agents de diagnostic, d'un anti-inflammatoire, d'un antimicrobien, d'un antibiotique, d'un facteur de croissance, d'une enzyme.

Dans la présente, le groupement Z peut être avantageusement un acide gras saturé ou insaturé. Il peut s'agir par exemple d'un acide gras choisi dans le groupe comprenant l'acide acétique, l'acide caprylique, l'acide caprique, l'acide laurique, l'acide myristique, l'acide palmitique, l'acide stéarique, l'acide arachidique, l'acide béhénique, l'acide lignocérique, l'acide cérotique, l'acide myristoléique, l'acide palmitoléique, l'acide sapiénique, l'acide oléique, l'acide élaïdique, l'acide trans-vaccénique, l'acide linoléique, l'acide linolélaïdique, l'acide α-linolénique, l'acide γ-linolénique, l'acide dihomo-γ-linolénique, l'acide arachidonique, l'acide eicosapentaénoïque, l'acide clupanodonique ou l'acide docosahexaénoïque. De préférence, l'acide gras est l'acide acétique.

Dans la présente, le groupement Z peut être avantageusement un acide aminé de la série L ou D choisi dans le groupe comprenant l'alanine, l'asparagine, une chaine aromatique par exemple la tyrosine, la phénylalanine, le tryptophane, la thyroxine ou l'histidine.

Avantageusement, les groupements Z peuvent conférer aux polymères des propriétés biologiques ou physicochimiques supplémentaires. Par exemple les groupements Z peuvent augmenter la solubilité ou la lipophilie dudit polymère permettant par exemple une meilleure diffusion ou pénétration tissulaire, par exemple l'augmentation de l'amphiphilie peut permettre une facilitation du franchissement de la barrière hématoencéphalique.

Des polymères dans lesquels Z est présent répondent à la formule Il suivante :

Aa Xx Yy Zz

dans laquelle, A, X, Y, a, x, y sont tel que défini ci-dessus et z représente le taux de substitution par des groupements Z.

Dans la présente le taux de substitution par des groupements Z représenté par « z » peut être compris de 0 à 50%, de préférence égale à 30%.

Les groupements X, Y et Z peuvent être indépendamment fixés sur le monomère A et/ou indépendamment fixés les uns aux autres. Lorsqu'au moins un des groupements X, Y et Z est indépendamment fixé sur un groupement X, Y et Z différent du premier, un desdits groupements X, Y ou Z est fixé au monomère A.

Ainsi, les groupements Z peuvent être fixés par covalence directement sur les monomères A ou fixés par covalence sur les groupements X et/ou Y.

Dans la présente, la composition peut comprendre une concentration de 0,01 microgramme à 100 mg en poids de polymère biocompatible par rapport au poids total de la composition. Par exemple la composition peut comprendre de 10 microgrammes à 10 milligrammes en poids par rapport au poids total de la composition.

Dans la présente, la concentration du polymère biocompatible dans la composition et/ou posologie d'administration de la composition peut être fonction de la voie d'administration envisagée pour la composition selon l'invention.

Par exemple, pour une administration par voie intracrânienne, il peut s'agir d'une administration unique de 1 à 5 mL, ou par minipompe, par exemple sur plusieurs jours. Par exemple la composition peut comprendre une concentration de 0,001 à 1 mg.mL⁻¹ de polymère biocompatible.

Dans la présente par « cellule eucaryote » on entend toute cellule eucaryote connue de l'Homme du métier. Il peut s'agir par exemple d'une cellule eucaryote de mammifère, par exemple une cellule eucaryote animale ou humaine. Il peut s'agir par exemple de toute cellule eucaryote quelque soit son stade de différenciation, par exemple une cellule choisie dans le groupe comprenant les cellules eucaryotes adultes ou embryonnaires, les cellules souches embryonnaires, les cellules souches adultes. Il peut s'agir par exemple de cellules eucaryotes de sang de cordon, de cellules de la moelle osseuse, de cellules du tissu adipeux, de cellules mésenchymateuses.

Il peut s'agir également d'une cellule souche pluri ou totipotente, ou de cellules engagées dans des voies de différenciations, par exemple des cellules souches mésenchymateuses. Il peut s'agir également d'une cellule souche pluri ou totipotente à l'exception des cellules souches embryonnaires.

Il peut s'agir par exemple d'une cellule hétérologue, homologue ou autologue d'un individu. De préférence, les cellules sont des cellules autologues.

Avantageusement, lorsque les cellules sont autologues, la composition selon l'invention la présente invention peut être préférée pour des raisons réglementaires, de sécurité, de faisabilité, d'efficacité et économique.

Avantageusement, lorsque les cellules sont autologues, elles sont de préférence isolées de l'individu et utilisées dans la composition selon l'invention et/ou utilisées dans un traitement dans les 24 heures après prélèvement et isolement sans autres ajouts. Avantageusement, cette administration unique permet de surmonter et de se conformer aux exigences/contraintes réglementaires.

Dans la présente la quantité de cellules comprises dans la composition peut être de 1 à 5.10⁷ cellules.

Dans la présente, par « composition pharmaceutique » on entend toute forme de composition pharmaceutique connue de l'Homme du métier. Dans la présente, la composition pharmaceutique peut être par exemple une solution injectable. Il peut s'agir par exemple une solution injectable, par exemple pour une injection locale ou systémique, par exemple en sérum physiologique, en solution glucose injectable, en présence d'excipients, par exemple de Dextrans, par exemple a des concentrations connues de l'homme du métier, par exemple du microgramme à quelques milligrammes par mL. La composition pharmaceutique peut être par exemple un médicament destiné à une administration orale choisie dans le groupe comprenant une formulation liquide, une forme posologique effervescente orale, une poudre orale, un système multiparticule, une forme galénique orodispersible.

Par exemple, lorsque la composition pharmaceutique est pour administration orale, elle peut être sous la forme d'une formulation liquide choisie dans le groupe comprenant une solution, un sirop, une suspension, une émulsion. Lorsque la composition pharmaceutique est sous la forme d'une forme posologique effervescente orale, elle peut être sous une forme choisie dans le groupe comprenant les comprimés, les granules, les poudres. Lorsque la composition pharmaceutique est sous la forme d'une poudre orale ou un système multiparticulaire, il peut être sous une forme choisie dans le groupe comprenant des billes, des granulés, des mini-comprimés et les microgranules. Lorsque la composition pharmaceutique est sous la forme d'une forme posologique orodispersible, elle peut être sous une forme choisie dans le groupe comprenant des comprimés orodispersibles, gaufrettes lyophilisées, films minces, un comprimé à mâcher, d'un comprimé, d'une capsule ou d'une gomme médicale à mâcher.

Selon la présente invention, la composition pharmaceutique peut être une composition pharmaceutique pour administration par voir orale, par exemple buccale et/ou sublingual, par exemple choisie dans le groupe comprenant les comprimés buccaux ou sublinguaux, les pastilles, gouttes, une solution pour pulvérisations.

Selon la présente invention, la composition pharmaceutique peut être une composition pharmaceutique pour administration topique, transdermique, par exemple choisie dans le groupe comprenant les pommades, crèmes, gels, lotions, patchs et mousses.

Selon la présente invention, la composition pharmaceutique peut être une composition pharmaceutique pour administration nasale, par exemple choisi dans le groupe comprenant des gouttes nasales, spray nasal, de la poudre nasale.

Selon la présente invention, la composition pharmaceutique peut être une composition pharmaceutique pour administration parentérale, par exemple sous-cutanée, intramusculaire, intraveineuse, intrathécale.

Dans la présente, la composition peut être formulée et/ou adaptée selon son administration. Par exemple, pour une administration par voie intraveineuse ou par voie intramusculaire la composition peut être administrée afin de délivrer une dose de polymère biocompatible comprise de 0,1 à 5 mg par kilogramme de poids corporel, ou pour une administration par voie orale la composition peut être administrée, par exemple en 2 à 5 prises égales par jour à hauteur d'un total quotidien par exemple de 15 à 500 mg de polymère biocompatible, ou pour une administration par voie intracrânienne la composition peut comprendre une concentration de 0,001 à 1 mg.ml⁻¹de polymère biocompatible, ou pour une administration par voie sublinguale la composition peut comprendre une concentration de 1 à 100mg/ml de polymère biocompatible, ou par voie aérienne la composition peut être administrée afin délivrer une dose comprise de 0,1 à 5 mg de polymère biocompatible par kilogramme de poids corporel dudit polymère.

La composition de la présente invention peut également comprendre au moins un autre ingrédient actif, particulièrement un autre ingrédient thérapeutiquement actif, par exemple pour une utilisation simultanée, séparée ou étalée dans le temps suivant la formulation galénique utilisée. Cet autre ingrédient peut être par exemple un ingrédient actif utilisé par exemple dans le traitement de maladies opportunes qui peuvent se développer chez un patient présentant une lésion tissulaire du système nerveux central. Il peut s'agir également de produits pharmaceutiques connus de l'homme du métier, par exemple des antibiotiques, anti inflammatoires , anticoagulants, des facteurs de croissance, des extraits plaquetaires, des neuroprotecteurs ou encore antidepresseurs, anticholesterols comme des statines etc.

Dans la présente, l'administration du polymère biocompatible et de la cellule peut être simultanée, successive ou concomitante.

Selon l'invention, au moins une des administrations peut être réalisée par voie orale ou par injection. Les deux administrations peuvent être réalisées de la même manière ou différemment. Par exemple, au moins une des administrations peut être réalisée par voie orale ou par injection. Par exemple, l'administration du polymère biocompatible et des cellules peut être faite par injection, l'administration du polymère biocompatible peut être par voie orale et les cellules peut être faite par injection systémique ou injection locale. L'administration peut être également fonction du site de la lésion.

Selon l'invention, l'utilisation de cellule eucaryote, notamment leur administration peut être réalisée dans un délai de 5 minutes à 3 mois, par exemple de 5 minutes à 1 semaine, de préférence de 5 minutes à 24 heures après la première administration dudit polymère biocompatible.

Selon l'invention, la composition peut être, par exemple, administrée quotidiennement, biquotidiennement et hebdomadairement. Il peut s'agir par exemple d'une administration une fois par jour, deux fois par jour ou plus.

Selon l'invention, la composition peut être, par exemple, administrée sur une durée de 1 jour à 3 mois, par exemple pendant 2 mois. Par exemple, la composition peut être administrée sur une période de 3 mois avec une fréquence d'administration tous les 15 jours.

Selon l'invention, le biopolymère peut être, par exemple, administré sur une durée de 1 jour à 3 mois, par exemple pendant 2 mois, avec par exemple une fréquence de 1 fois par jour, et la cellule eucaryote administrée sur une durée identique ou différente, par exemple une durée de 1 jour à 3 mois, avec une fréquence de hebdomadaire

Selon l'invention, lorsque l'administration des polymères et des cellules sont successives, la posologie pour chaque administration peut être une administration des polymères suivie de l'administration des cellules. Par exemple, les cellules peuvent être administrées de 1 minute à 24 heures après l'administration des polymères, de 30 minutes à 12 heures après administration des polymères, de 45 minutes à 6 heures après administration des polymères, 1 heure après administration des polymères.

La présente invention se rapporte également à un procédé de traitement d'un patient ayant subi une ischémie cérébrale comprenant dans un ordre quelconque les étapes suivantes :
i.l'administration d'au moins un polymère biocompatible, et
ii. l'administration d'au moins une cellule eucaryote,
dans lequel, les administrations sont concomitantes, successives ou alternatives.

Le polymère biocompatible est tel que défini ci-dessus.

La cellule eucaryote est telle que définie ci-dessus.

Selon l'invention, le patient peut être tout mammifère. Il peut s'agir par exemple d'un animal ou d'un être humain.

Selon l'invention, la cellule eucaryote administrée peut être une cellule hétérologue ou homologue du dit patient.

Selon l'invention, le mode et/ou la voie d'administration du polymère biocompatible peut être tel(s) que défini(s) ci-dessus.

Selon l'invention, le mode et/ou la voie d'administration de la cellule peut être tel(s) que défini(s) ci-dessus.

Selon l'invention la fréquence d'administration du polymère biocompatible peut être telle que définie ci-dessus.

Selon l'invention la fréquence d'administration de la cellule eucaryote peut être telle que définie ci-dessus.

Selon l'invention, lorsque l'administration des polymères biocompatibles et des cellules sont successives, la posologie pour chaque administration peut être une administration des polymères biocompatibles suivie de l'administration des cellules. Par exemple, les cellules peuvent être administrées de 1 minute à 48 heures après l'administration des polymères biocompatibles, de 30 minutes à 12 heures après administration des polymères, de 45 minutes à 6 heures après administration des polymères, 1 heure après administration des polymères.

Avantageusement, la cellule eucaryote est une cellule souche adulte mésenchymateuse.

En d'autres termes, même si dans la présente description il est fait référence à une composition, on comprend bien que chacun des composés de la composition peut être administré concomitamment aux autres composés (par exemple dans une seule composition ou dans deux compositions, chacune de ces compositions comprenant un ou plusieurs des composants précités, le mode d'administration de chacun des composés ou composition(s) pouvant être identique ou différent), ou indépendamment les un des autres, par exemple successivement, par exemple administration indépendante d'un polymère biocompatible et, administration indépendante d'une cellule eucaryote, ces administrations étant réalisées sur un même patient, concomitamment ou successivement ou alternativement, dans un ordre qui est celui précité ou un autre ordre. Ces différentes administrations peuvent être réalisées, indépendamment l'une de l'autre ou de manière liée (composition ou co-administration), par un mode d'administration identique ou différent (injection, ingestion, application topique, etc.), une ou plusieurs fois par jour, pendant un ou plusieurs jours successifs ou non.

La présente invention a également pour objet un kit pharmaceutique pour la prévention et/ou le traitement de lésions tissulaires du système nerveux central dues à une ischémie vasculaire cérébrale comprenant :
i. un polymère biocompatible, et
ii. au moins une cellule eucaryote.

Le polymère biocompatible est tel que défini ci-dessus.

La cellule eucaryote est telle que définie ci-dessus.

La présente invention a également pour objet l'utilisation d'une composition pharmaceutique comprenant
i. un polymère biocompatible, et
ii. au moins une cellule eucaryote
pour la fabrication d'un médicament pour le traitement de lésions tissulaires du système nerveux central dues à une ischémie vasculaire cérébrale.

Le polymère biocompatible est tel que défini ci-dessus.

La cellule eucaryote est telle que définie ci-dessus.

Dans ce mode de réalisation, par médicament on entend une composition pharmaceutique telle que définie ci-dessus.

Les inventeurs ont démontré de manière surprenante et inattendue que la composition selon l'invention, permet avantageusement une diminution significative de lésions ischémiques.

En outre, les inventeurs ont également démontré que la composition selon l'invention permet avantageusement une récupération fonctionnelle précoce et durable post-ischémique.

Les inventeurs ont également démontré que la composition selon l'invention permet avantageusement une amélioration précoce des fonctions neurologiques, des performances sensori-motrices après administration de la composition selon l'invention.

De plus, les inventeurs ont également démontré que la composition selon l'invention permet avantageusement de limiter/réduire le volume d'infarctus dus par exemple à une lésion tissulaire liée par exemple à un accident vasculaire cérébral.

En outre, les inventeurs ont également démontré que la composition selon l'invention permet avantageusement de protéger et/ou stimuler la régénération du tissu cérébral présentant des lésions liée par exemple à un accident vasculaire cérébral et/ou un traitement par radiothérapie.

D'autres avantages pourront encore apparaître à l'Homme du métier à la lecture des exemples ci-dessous, illustrés par les figures annexées, donnés à titre illustratif.

### Brève description des figures

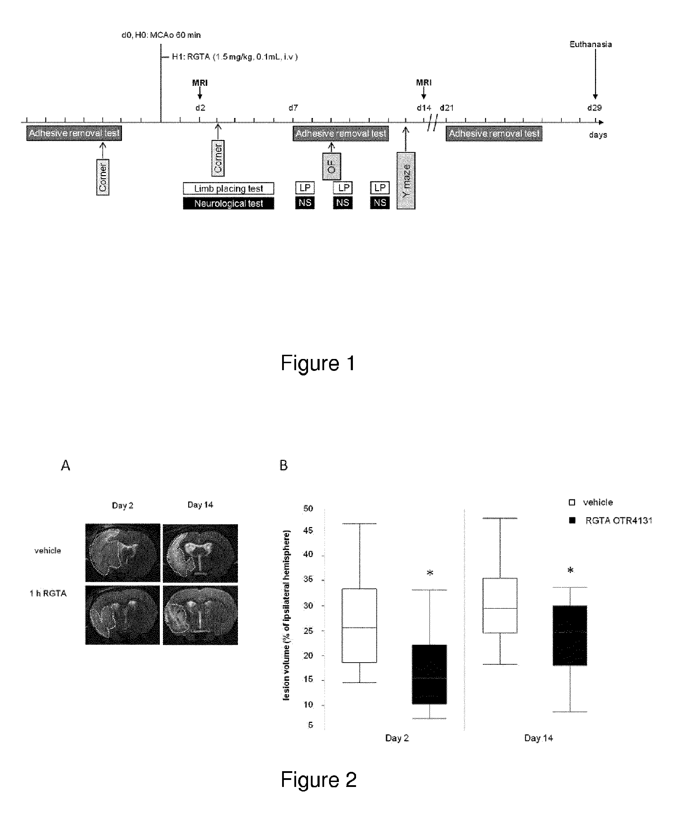

- La figure 1 représente un schéma du protocole expérimental visant à étudier les effets d'un polymère biocompatible sur les dommages cérébraux et les déficits neurologiques. Sur cette figure MCAo signifie occlusion de l'artère cérébrale moyenne (« middle cerebral artery occlusion »), LP signifie test du placement de pattes (« limb placing test »); NS signifie score neurologique (« neurological score ») ; OF signifie champs ouvert (« open field »); MRI signifie imagerie par résonnance magnétique (« magnetic resonance imaging »).
- La figure 2 représente des photographies du système nerveux central (cerveau) (figure 2 A) représentant un infarctus (zone entre pointillées) sans application (1) ou après application (2) d'un polymère biocompatible après deux jours (J2) ou quatorze jours (J14) de l'évènement ischémique inducteur de la lésion. La figure 2 B représente un diagramme représentant le volume de la lésion (ordonnée) en fonction du jour (abscisse) sans (bâtons blancs) ou avec application d'un polymère biocompatible (bâtons noirs).

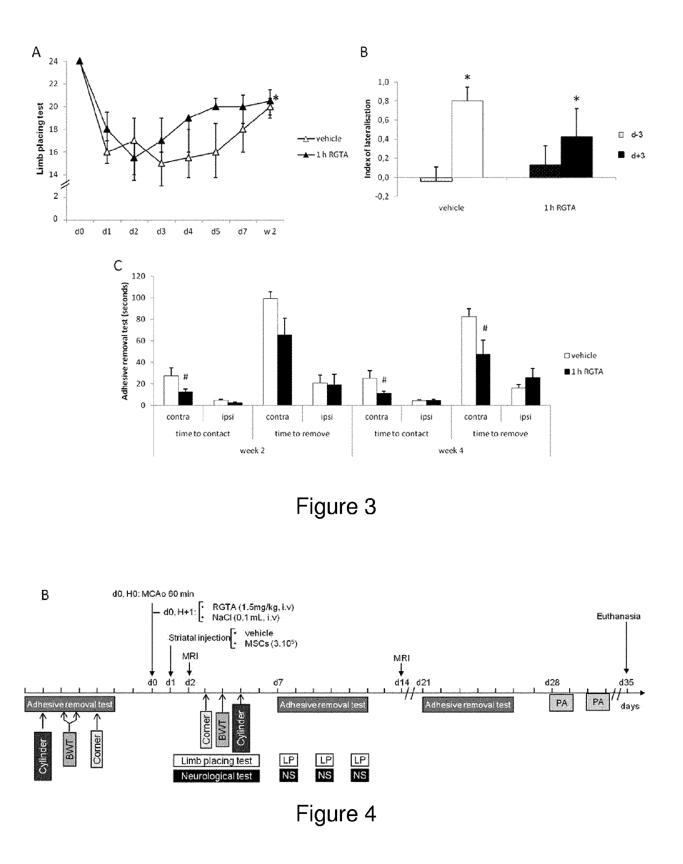

- La figure 3 représente un diagramme (figure 3 A) représentant les résultats du test du placement de pattes (*ANOVA à mesure répétée p<0.05) en fonction du temps après administration (triangles pleins) ou non (triangles vides) d'un polymère biocompatible. La figure 3 B représente un diagramme en bâton des résultats de latéralisation évaluée à l'aide du test du coin (* comparaison de la moyenne à la valeur référence 0 p<0.05) à plus ou moins trois jours après administration (bâtons pleins) ou non (bâtons vides) d'un polymère biocompatible. La figure 3 C représente un diagramme en bâtons de l'évaluation de la récupération sensitivo-motrice fine à l'aide du test du retrait de l'adhésif (*p<0.05, ANOVA 1 facteur) après 2 ou 4 semaines après administration (bâtons pleins) ou non (bâtons vides) d'un polymère biocompatible, l'ordonné représentant le temps en secondes.
- La figure 4 représente un schéma du protocole expérimental visant à étudier les effets d'une co-administration d'un polymère biocompatible et de cellules souches adultes (cellules souches mésenchymateuses) via une étude IRM associée à des tests comportementaux à savoir le BWT (« beam walking test ») test de la poutre; LP (« limb placing test ») test du placement de pattes; NS (« neurological score ») score neurologique et le PA (« passive avoidance ») test de l'évitement passif.

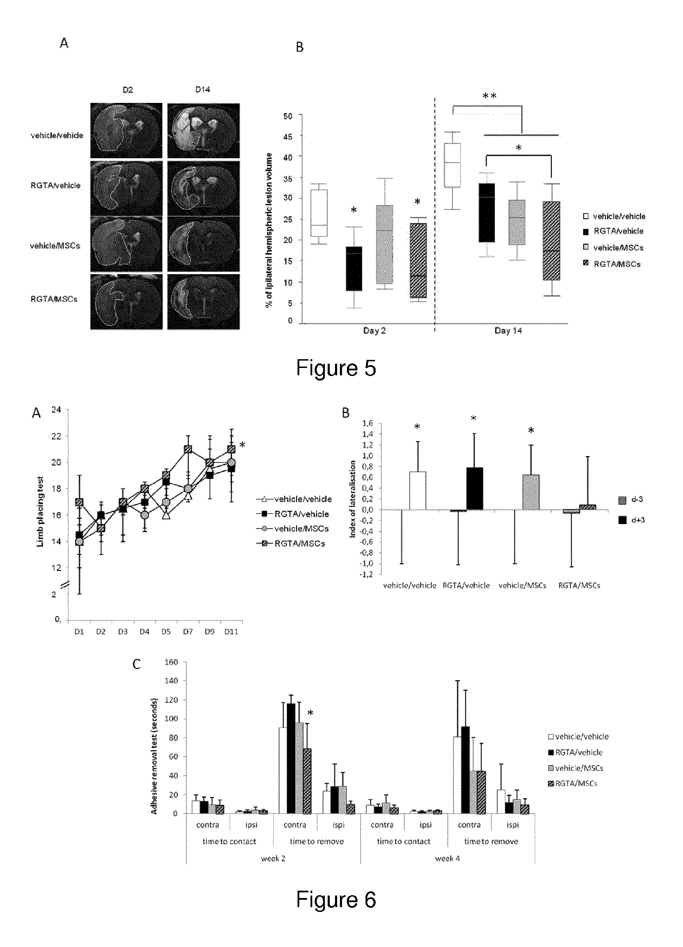

- La figure 5 représente des photographies du système nerveux central (cerveau) (figure 5 A) représentant un infarctus (zone entre pointillées) sans application (1) ou après application (2) d'un polymère biocompatible, après application de cellules souches mésenchymateuses (3) et après application d'un polymère biocompatible et de cellules souches mésenchymateuses (4) à deux jours (J2) ou quatorze jours (J14) de l'évènement ischémique inducteur de la lésion. La figure 5 B représente un diagramme représentant le volume de la lésion (ordonnée) en fonction du jour (abscisse) sans (bâtons blancs) avec application d'un polymère biocompatible (bâtons noirs), avec application de cellules souches mésenchymateuses (bâtons hachurés horizontalement) ou après application d'un polymère biocompatible et de cellules souches mésenchymateuses (bâtons hachurés diagonalement)
- La figure 6 représente un diagramme (figure 6 A) représentant les résultats du test du placement de pattes (*ANOVA à mesure répétée p<0.05) en fonction du temps après administration (carrés pleins) ou non (triangles vides) d'un polymère biocompatible, après administration de cellules mésenchymateuses (ronds pleins) et d'un polymère biocompatible et de cellules mésenchymateuses (carrés hachurés). La figure 6 B représente un diagramme en bâton des résultats de latéralisation évaluée à l'aide du test du coin (* comparaison de la moyenne à la valeur référence 0 p<0.05) à plus ou moins trois jours après administration (bâtons pleins) ou non (bâtons vides) d'un polymère biocompatible, administration de cellules souches mésenchymateuses et administration de cellules souches mésenchymateuses et d'un polymère biocompatible. La figure 6 C représente un diagramme en bâtons de l'évaluation de la récupération sensitivo-motrice fine à l'aide du test du retrait de l'adhésif (*p<0.05, ANOVA 1 facteur) après 2 ou 4 semaines après administration (bâtons pleins) ou non (bâtons vides) d'un polymère biocompatible, avec application de cellules souches mésenchymateuses (bâtons hachurés horizontalement) ou après application d'un polymère biocompatible et de cellules souches mésenchymateuses (bâtons hachurés diagonalement), l'ordonné représentant le temps en secondes.

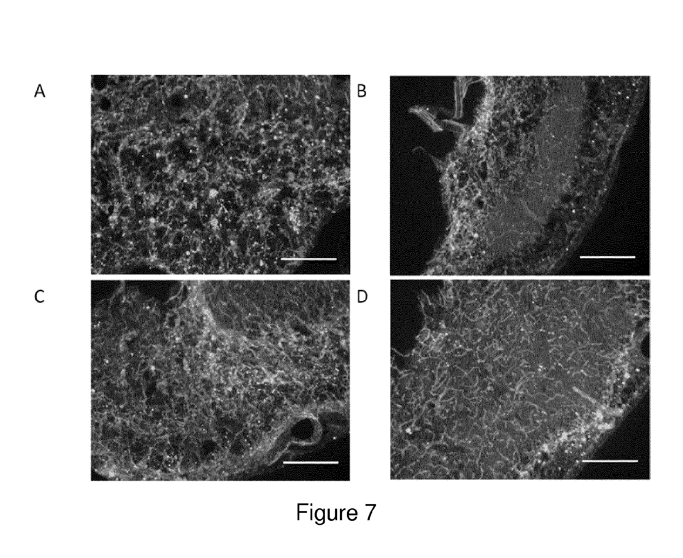

- La figure 7 représente des photographies en microscopie optique de la vascularisation dans la zone ischémique 35 jours après occlusion de l'artère cérébrale moyenne dans les groupes véhicule/véhicule (A), véhicule/cellules souches mésenchymateuses (B), polymère biocompatible/véhicule (C) et d'un polymère biocompatible / cellules souches mésenchymateuses (D). L'échelle est de 500µm.

### EXEMPLES

### Exemple 1 : Evaluation de l'effet d'un polymère biocompatible selon l'invention sur des dommages cérébraux et des déficits fonctionnels dus à une ischémie cérébrale

Dans cet exemple, le polymère biocompatible était le polymère commercialisé par la société OTR3 sous la référence commerciale OTR 4131 décrit dans Frescaline G. et al., Tissue Eng Part A. 2013 Jul;19(13-14):1641-53. doi: 10.1089/ten.TEA.2012.0377 disponible dans le commerce.

Les rats étaient des rats males de la souche Sprague Dawley.

Afin de définir les effets du polymère biocompatible OTR 4131 sur les dommages cérébraux et les déficits fonctionnels, le protocole expérimental illustré dans la figure 1 a été réalisé chez des rats soumis à une ischémie cérébrale transitoire par occlusion de l'artère cérébrale moyenne.

En particulier, L'animal a été anesthésié par inhalation d'isoflurane 5 % dans un mélange d'O₂/N₂O dans des proportions respective de 1/3 pendant 3 minutes, puis maintenu à l'aide de 2-2,5 % d'isoflurane délivré à l'aide d'un masque le temps de la chirurgie. Le rat a été placé en décubitus dorsal. Une incision a été pratiquée au niveau du cou. Les artères carotide commune, carotide externe et carotide interne ont été isolées puis un filament occlusif a été introduit dans la carotide externe a été avancé jusqu'à la partie proximal de l'artère cérébrale moyenne. Une heure après, le filament a été retiré pour permettre la reperfusion, par exemple tel que décrit dans Letourneur et al., 2011 « Impact of genetic and renovascular chronic arterial hypertension on the acute spatiotemporal évolution of the ischemic penumbra: a sequential study with MRI in the rat » J Cereb Blood Flow Metab. 2011 Feb;31(2):504-13 ou encore Quittet et al., « Effects of mesenchymal stem cell therapy, in association with pharmacologically active microcarriers releasing VEGF, in an ischaemic stroke model in the rat. » Acta Biomater. 2015 Mar;15:77-88.

Une heure après l'induction de l'ischémie, 1.5 mg/kg de OTR 4131 ont été administrés par voie intraveineuse, et ensuite l'animal a été réveillé

Afin d'évaluer les effets du traitement sur le volume ischémique, une étude IRM (imagerie par résonnance magnétique (7T, PharmaScan, Bruker BioSpin, Ettlingen, Germany)) a été réalisée à 48h et à 14 jours après l'induction de l'ischémie cérébrale. Pour ce faire, l'animal a été anesthésié par inhalation d'isoflurane 5 % dans un mélange d'O₂ / N₂O 1/3 pendant 3 minutes puis maintenu anesthésié avec 2-2,5 % d'isoflurane. Une séquence T2 anatomique a été utilisée selon un mode d'acquisition rapide RARE 8 par écho de spin rapide (RARE rapid acquisition with refocused echoes) avec un temps de répétition de 5000 millisecondes, un temps d'écho de 16,25 millisecondes, un moyennage (NEX number of experiments) = 2, une matrice de 256 x 256 pixels et une taille d'image ou FOV (field of view) de 3,84 x 3,84 cm soit une résolution nominale de 0,15 x 0,15 x 0,75 mm³. Vingt coupes contiguës ont été réalisées par animal avec un temps total d'acquisition de 4 minutes.

La figure 2A représente les images IRM obtenues après 2 ou 14 jours post ischémie cérébrale transitoire. Tel que démontré sur cette figure, une diminution de l'infarctus a été observé après une injection 1 heure après le début de l'ischémie du polymère biocompatible (zone entourée en pointillé) par rapport au rat n'ayant pas reçu de polymère biocompatible. Une diminution significative du volume d'infarctus est observée à J2 et à J14 lorsque le traitement est administré 1 h post-occlusion (Figure 2).

Cette expérience a été également réalisé en changeant le temps d'injection : injection à 2h30 ou à 6h après l'induction de l'ischémie cérébrale et a montré une absence de résultats significatifs (résultats non fournis). En d'autres termes une injection seule du polymère biocompatible 2h30 ou 6h après induction de l'ischémie n'a pas d'effet sur l'infarctus provoqué par l'ischémie.

Une évaluation de l'effet du polymère biocompatible sur la récupération fonctionnelle a été également réalisée. Pour ce faire, une batterie de tests sensori-moteurs et cognitifs a été réalisée selon le procédé décrit dans Quittet et al., « Effects of mesenchymal stem cell therapy, in association with pharmacologically active microcarriers releasing VEGF, in an ischaemic stroke model in the rat. » Acta Biomater. 2015 Mar;15:77-88 ou Freret et al., 2006 "Long-term functional outcome following transient middle cérébral artery occlusion in the rat: correlation between brain damage and behavioral impairment." Behav Neurosci. 2006 Dec;120(6):1285-98.

Les résultats obtenus sont représentés sur la figure 3. Tel que démontré sur cette figure l'injection du polymère biocompatible 1h après l'induction de l'ischémie permet une amélioration de la récupération fonctionnelle, par exemple comme démontré dans le test du placement de pattes, évaluant les performances sensitives (ANOVA mesure répétée p<0.05) (figure 3A triangle plein) par rapport au rat n'ayant pas reçu d'injection, mais également dans le test du coin évaluant la latéralisation des animaux via la comparaison de la moyenne à la valeur référence p>0,05 (Figure 3 B bâtons pleins) par rapport au rat n'ayant pas reçu d'injection.

En outre, une évaluation fine plus tardive des performances sensitivo-motrices a été réalisée grâce au test du retrait de l'adhésif. Les résultats obtenus sont illustrés sur la figure 3 C. Tel que démontré sur cette figure, les animaux ayant reçu une administration de polymère biocompatible 1h post-occlusion (bâtons pleins) ont tendance à détecter la présence de l'adhésif du côté controlésionnel touché par l'ischémie plus rapidement que les autres groupes n'ayant pas reçu de polymère biocompatible à la semaine 2 (ANOVA 1 facteur p=0.1). La répétition du test à la semaine 4 a mis en évidence une pérennité de la tendance concernant la détection de l'adhésif du côté controlésionnel (ANOVA 1 facteur p=0.1). A cette dernière, s'est ajouté un retrait significativement plus rapide de l'adhésif du côté controlésionnel pour les animaux traités avec le polymère biocompatible témoignant d'une amélioration plus rapide des performances sensitivo-motrices induite par le polymère biocompatible.

### Exemple 2 : Evaluation de l'effet de la co-administration d'un polymère biocompatible et d'une cellule souche mésenchymateuse sur des dommages cérébraux et des déficits fonctionnels dus à un choc ischémique

Dans cet exemple, les rats et le polymère biocompatible étaient identiques à ceux de l'exemple 1

Les cellules souches mésenchymateuses ont été extraites des fémurs et tibia de rats Sprague Dawley selon la méthode décrite dans le document Quittet et al. « Effects of mesenchymal stem cell therapy, in association with pharmacologically active microcarriers releasing VEGF, in an ischaemic stroke model in the rat » Acta Biomater. 2015 Mar;15:77-88.

Afin de définir les effets de la co-administration du polymère biocompatible OTR 4131 et des cellules souches mésenchymateuses sur les dommages cérébraux et les déficits fonctionnels, le protocole expérimental illustré dans la figure 4 a été réalisé chez des rats soumis à une ischémie cérébrale transitoire par occlusion de l'artère cérébrale moyenne selon l'approche intraluminale telle que décrite dans l'exemple 1 ci-dessus.

Une évaluation de effets de la co-administration sur le volume d'infarctus mais également sur la récupération fonctionnelle post-ischémique a été réalisée.

Afin d'évaluer les effets du traitement sur le volume ischémique, une étude IRM (imagerie par résonnance magnétique) a été réalisée à 48h et à 14 jours après l'induction de l'ischémie cérébrale comme décrit dans l'exemple 1 ci-dessus.

L'analyse IRM au bout de 48 heures a révélé une diminution du volume d'infarctus par rapport au groupe contrôle pour les animaux traités avec le RGTA et la co-administration RGTA-MSCs (ANOVA 1 facteur, p<0.05) comme illustré sur la figure 5 A (zone entre pointillé). En particulier, il a été clairement démontré que la co-administration permet avantageusement une diminution du volume de la lésion à 48 heures par rapport au sujet n'ayant pas reçu d'injection mais permet également de manière surprenante au bout de 14 jours de réduire de manière significative le volume de la lésion par rapport notamment aux animaux traités avec le polymère biocompatible seul, les MSCs seules (Figure 5 A et B). Ainsi, cette expérience démontre clairement que la composition selon l'invention et/ou l'administration du polymère biocompatible et de la cellule permet d'obtenir un nouvel effet technique non observé en leur absence et/ou lors de leur seule administration.

Une évaluation de l'effet de la co-administration du polymère biocompatible et des cellules souches sur la récupération fonctionnelle a été également réalisée. Pour ce faire, une batterie de tests sensori-moteurs et cognitifs a été réalisée.

Les résultats obtenus sont représentés sur la figure 6. Tel que démontré sur cette figure, les effets du traitement sur les performances sensori-motrices et cognitives, l'évaluation de la récupération sensitive précoce à l'aide du test du placement de pattes (figure 6 A) a mis en évidence une meilleure récupération pour les animaux du groupe polymère biocompatible-cellules souches mésenchymateuses (carrés hachurés) par rapport aux trois autres groupes (ANOVA à mesure répétée p<0.05).

Concernant, la latéralisation qui a été évaluée à l'aide du test du coin, il a été également mis en exergue une potentialisation de la récupération fonctionnelle démontrée par l'indice de latéralisation non différent de la valeur référence fixée à 0 (valeur de non latéralisation) uniquement pour les animaux du groupe polymère biocompatible-cellules souches mésenchymateuses (figure 6B). Enfin, concernant le test du retrait de l'adhésif, il a été démontré que ce dernier était accéléré dès la seconde semaine post-occlusion dans le côté controlésionnel touché par l'ischémie uniquement pour les animaux du groupe polymère biocompatible-cellules souches mésenchymateuses (ANOVA 1 facteur p<0.05) (Figure 6C bâtons hachurés).

Ces résultats démontrent donc clairement que l'association du polymère biocompatible et des cellules eucaryotes, en particuliers les cellules souches mésenchymateuses (MSCs) permet d'obtenir et de traiter les lésions tissulaires du système nerveux central. En particulier, elle permet également et de manière surprenante une récupération fonctionnelle très supérieure à celle des animaux non traités mais également très supérieure à celle des animaux traités uniquement avec le polymère biocompatible ou les MSCs seuls.

Une évaluation ex-vivo a été également réalisée. Pour ce faire, les coupes du cerveau ont été rincées à 3 reprises pendant 5 minutes dans du PBS 0,1 M puis ont été incubées dans un mélange de PBS 0,1 M / 3 % BSA (bovine serum albumine Sigma^{®}) / 0,1 % triton (Sigma^{®}) pendant au moins 1 heure afin de saturer les sites de liaison non spécifiques. Les coupes ont été par la suite placées en contact avec l'anticorps primaire (RECA-1 ; AbDSerotec, dilué à 1 :100 dans du PBS 0,1 M / BSA 1 % / triton 0,1 % toute la nuit à 4 °C sous agitation douce. Les coupes ont été par la suite rincées à 3 reprises avec du PBS 0,1 M puis incubées 2 heures avec l'anticorps secondaire dilué dans une solution de 0,1 M PBS / 1 % BSA / 0,1 % triton. Les coupes ont été rincées à 3 reprises dans du PBS avant d'être montées entre lame et lamelle. Des photos ont été acquises à l'aide d'un microscope droit équipé d'une caméra et du logiciel MétaVue. Les images ainsi obtenues ont été analysées à l'aide du logiciel ImageJ (http://imagej.nih.gov/ij/).

Ainsi, la vascularisation du tissu ischémié a été évaluée par immunofluorescence à l'aide d'un marquage des cellules endothéliales par l'anticorps RECA-1 (Rat Endothelial Cell Antibody-1). Le marquage a permis le cas échéant d'identifier et de mettre en avant l'architecture vasculaire du tissu représenté par les traits blancs dans les zones grisées.

Tel que démontré sur la figure 7 représentant les photographies en microscopie électronique obtenu, le marquage révèle qu'en l'absence de polymère biocompatible ou de la combinaison du polymère biocompatible et des cellules souches mésenchymateuses aucune préservation de l'architecture de la vascularisation dans la zone d'infarctus n'a été observée (figure 7 A et C). Seul en présence de polymère biocompatible (figure 7 B) ou de la combinaison du polymère biocompatible et des cellules souches mésenchymateuses (figure 7 D) une préservation de la structure vasculaire a pu être observée. En outre, la figure 7 D démontre clairement que combinaison du polymère biocompatible et des cellules souches mésenchymateuses permet d'obtenir un effet surprenant et inattendue sur cette préservation de la structure vasculaire.

Cet exemple démontre donc clairement que la composition selon l'invention permet avantageusement de prévenir et/ou de traiter les lésions tissulaires du système nerveux central dues à une ischémie vasculaire cérébrale. En outre, cet exemple démontre clairement que la composition selon l'invention permet en outre de traiter les éventuels déficits fonctionnels dus aux lésions tissulaires du système nerveux central. En outre, cet exemple démontre clairement que la composition selon l'invention permet avantageusement de diminuer le temps de récupération et/ou de permettre une récupération des éventuels déficits fonctionnels dus à la lésion tissulaire.

Cet exemple démontre donc clairement que la composition selon l'invention présente des effets bénéfiques important dans l'ischémie à la fois en terme de protection tissulaire, par exemple en limitant le volume de l'infarctus, mais également en termes de récupération fonctionnelle comme illustré précédemment. A ces effets bénéfiques s'ajoute en outre une amélioration de la préservation de l'architecture du système vasculaire dans la zone d'infarctus.

### Listes des références

1. Adeoye, O., Hornung, R., Khatri, P., & Kleindorfer, D. (2011). Recombinant tissue-type plasminogen activator use for ischemic stroke in the United States: a doubling of treatment rates over the course of 5 years. Stroke; a Journal of Cerebral Circulation, 42(7), 1952-5.
2. Aggarwal, S., & Pittenger, M. F. (2005). Human mesenchymal stem cells modulate allogeneic immune cell responses. Blood, 105(4), 1815-22.
3. Altman, J., & Das, G. D. (1965). Autoradiographic and histological evidence of postnatal hippocampal neurogenesis in rats. The Journal of Comparative Neurology, 124(3), 319-35.
4. Andres, R. H., Horie, N., Slikker, W., Keren-Gill, H., Zhan, K., Sun, G., Steinberg, G. K. (2011). Human neural stem cells enhance structural plasticity and axonal transport in the ischaemic brain. Brain, 134(6), 1777-1789.
5. Bang, O. Y., Lee, J. S., Lee, P. H., & Lee, G. (2005). Autologous mesenchymal stem cell transplantation in stroke patients. Annals of Neurology, 57(6), 874-82.
6. Barritault, D., Garcia-Filipe, S., & Zakine, G. (2010). Basement of matrix therapy in regenerative medicine by RGTA(®): From fundamental to plastic surgery. Annales de Chirurgie Plastique et Esthetique, 55(5), 413-420.
7. Bhasin, A., Srivastava, M., Kumaran, S., Mohanty, S., Bhatia, R., Bose, S., Airan, B. (2011). Autologous mesenchymal stem cells in chronic stroke. Cerebrovascular Diseases Extra, 1(1), 93-104.
8. Cramer, S. C. (2008). Repairing the human brain after stroke: I. Mechanisms of spontaneous recovery. Annals of Neurology, 63(3), 272-287.
9. Crisan, M., Yap, S., Casteilla, L., Chen, C. W., Corselli, M., Park, T. S., Peault, B. (2008). A perivascular origin for mesenchymal stem cells in multiple human organs. Cell Stem.Cell, 3, 301-313.
10. Da Silva Meirelles, L., Chagastelles, P. C., & Nardi, N. B. (2006). Mesenchymal stem cells reside in virtually all post-natal organs and tissues. Journal of Cell Science, 119, 2204-2213.
11. Desgranges, P., Barbaud, C., Caruelle, J. P., Barritault, D., & Gautron, J. (1999). A substituted dextran enhances muscle fiber survival and regeneration in ischemic and denervated rat EDL muscle. The FASEB Journal, 13(6), 761-766.
12. Di Nicola, M. (2002). Human bone marrow stromal cells suppress T-lymphocyte proliferation induced by cellular or nonspecific mitogenic stimuli. Blood, 99(10), 3838-3843.
13. Eckert, M. a, Vu, Q., Xie, K., Yu, J., Liao, W., Cramer, S. C., & Zhao, W. (2013). Evidence for high translational potential of mesenchymal stromal cell therapy to improve recovery from ischemic stroke. Journal of Cerebral Blood Flow and Metabolism, 33(9), 1322-34.
14. Erices, A., Conget, P., & Minguell, J. J. (2000). Mesenchymal progenitor cells in human umbilical cord blood. British Journal of Haematology, 109(1), 235-42.
15.Esneault, E., Pacary, E., Eddi, D., Freret, T., Tixier, E., Toutain, J., ... Bernaudin, M. (2008). Combined therapeutic strategy using erythropoietin and mesenchymal stem cells potentiates neurogenesis after transient focal cerebral ischemia in rats. Journal of Cerebral Blood Flow and Metabolism 28(9), 1552-63.
16. Friedenstein, A., Chailakhjan, R., & Lalykina, K. (1970). The development of fibroblast colonies in monolayer cultures of guinea. Cell Proliferation, 3(4), 393-403.
17. Gage, F. H. (2002). Neurogenesis in the adult brain. The Journal of Neuroscience : The Official Journal of the Society for Neuroscience, 22(3), 612-3.
18. In 't Anker, P. S., Scherjon, S. a, Kleijburg-van der Keur, C., de Groot-Swings, G. M. J. S., Claas, F. H. J., Fibbe, W. E., & Kanhai, H. H. H. (2004). Isolation of mesenchymal stem cells of fetal or maternal origin from human placenta. Stem Cells, 22(7), 1338-45.
19. Jaillard, A., Naegele, B., Trabucco-Miguel, S., LeBas, J. F., & Hommel, M. (2009). Hidden dysfunctioning in subacute stroke. Stroke, 40(7), 2473-9.
20. Kranz, a, Wagner, D. C., Kamprad, M., Scholz, M., Schmidt, U. R., Nitzsche, F., Boltze, J. (2010). Transplantation of placenta-derived mesenchymal stromal cells upon experimental stroke in rats. Brain Research, 1315, 128-136.
21. Lee, J. S., Hong, J. M., Moon, G. J., Lee, P. H., Ahn, Y. H., & Bang, O. Y. (2010). A long-term follow-up study of intravenous autologous mesenchymal stem cell transplantation in patients with ischemic stroke. Stem Cells, 28(6), 1099-1106.
22. Lees, K. R., Bluhmki, E., von Kummer, R., Brott, T. G., Toni, D., Grotta, J. C., Hacke, W. (2010). Time to treatment with intravenous alteplase and outcome in stroke: an updated pooled analysis of ECASS, ATLANTIS, NINDS, and EPITHET trials. The Lancet, 375(9727), 1695-1703.
23. Malgieri, A., Kantzari, E., Patrizi, M. P., & Gambardella, S. (2010). Bone marrow and umbilical cord blood human mesenchymal stem cells: state of the art. International Journal of Clinical and Experimental Medicine, 3(4), 248-69.
24. Paul, G., & Anisimov, S. V. (2013). The secretome of mesenchymal stem cells: Potential implications for neuroregeneration. Biochimie, 95(12), 2246-2256.
25. Rouet, V., Hamma-Kourbali, Y., Petit, E., Panagopoulou, P., Katsoris, P., Barritault, D., Courty, J. (2005). A synthetic glycosaminoglycan mimetic binds vascular endothelial growth factor and modulates angiogenesis. The Journal of Biological Chemistry, 280(38), 32792-32800.
26. Seri, B., Herrera, D. G., Gritti, a, Ferron, S., Collado, L., Vescovi, a, ... Alvarez-Buylla, A. (2006). Composition and organization of the SCZ: a large germinal layer containing neural stem cells in the adult mammalian brain. Cerebral Cortex, 16 Suppl 1, i103-i111.
27.Song, H., Cha, M.-J., Song, B.-W., Kim, I.-K., Chang, W., Lim, S., ... Hwang, K.-C. (2010). Reactive oxygen species inhibit adhesion of mesenchymal stem cells implanted into ischemic myocardium via interference of focal adhesion complex. Stem Cells, 28(3), 555-63.
28. Song, H., Song, B.-W., Cha, M.-J., Choi, I.-G., & Hwang, K.-C. (2010). Modification of mesenchymal stem cells for cardiac regeneration. Expert Opinion on Biological Therapy, 10(3), 309-19.
29.Suárez-Monteagudo, C., Hernández-Ramirez, P., Alvarez-González, L., García-Maeso, I., de la Cuétara-Bernal, K., Castillo-Díaz, L., ... Bergado, J. (2009). Autologous bone marrow stem cell neurotransplantation in stroke patients. An open study. Restorative Neurology 27(3), 151-161.
30.Toma, C., Pittenger, M. F., Cahill, K. S., Byrne, B. J., & Kessler, P. D. (2002). Human mesenchymal stem cells differentiate to a cardiomyocyte phenotype in the adult murine heart. Circulation, 105(1), 93-98.
31. Wang, Y., Liu, J., Tan, X., Li, G., Gao, Y., Liu, X., Li, Y. (2013). Induced pluripotent stem cells from human hair follicle mesenchymal stem cells. Stem Cell Reviews, 9(4), 451-60.
32.Yalvac, M. E., Rizvanov, A. a, Kilic, E., Sahin, F., Mukhamedyarov, M. a, Islamov, R. R., & Palotás, A. (2009). Potential role of dental stem cells in the cellular therapy of cerebral ischemia. Current Pharmaceutical Design, 15(33), 3908-16.
33.Yamagata, M., Yamamoto, A., Kako, E., Kaneko, N., Matsubara, K., Sakai, K., Ueda, M. (2013). Human dental pulp-derived stem cells protect against hypoxic-ischemic brain injury in neonatal mice. Stroke; a Journal of Cerebral Circulation, 44(2), 551-4.
34.Yamauchi, H., Desgranges, P., Lecerf, L., Papy-Garcia, D., Tournaire, M. C., Moczar, M., Barritault, D. (2000). New agents for the treatment of infarcted myocardium. FASEB Journal : Official Publication of the Federation of American Societies for Experimental Biology, 14(14), 2133-4.

## Revendications

1. Composition pharmaceutique pour son application comme médicament pour la prévention et/ou le traitement de lésions tissulaires du système nerveux central dues à une pathologie hypoxique cérébrale, ladite composition comprenant
- un polymère biocompatible de formule générale (I) suivante
AaXxYy (I)
dans laquelle :
A représente un monomère,
X représente un groupement R₁COOR₂, ou - R₉(C=O)R₁₀ Y représente un groupement O ou N-sulfonate et répondant à l'une des formules suivante -R₃OSO₃R₄, -R₅NSO₃R₆, -R₇SO₃R₈ dans lesquelles :
R₁, R₃, R₅ et R₉ représentent indépendamment une chaîne hydrocarbonée aliphatique, éventuellement ramifiée et/ou insaturée et éventuellement qui contient un ou plusieurs cycles aromatiques,
R₂, R₄, R₆ et R₈ représentent indépendamment un atome d'hydrogène ou un cation M⁺, et
R₇ et R₁₀ représentent indépendamment une liaison, une chaîne hydrocarbonée aliphatique, éventuellement ramifiée et/ou insaturée, a représente le nombre de monomères,
x représente le taux de substitution des monomères A par des groupements X,
y représente le taux de substitution des monomères A par des groupements Y, et
- une cellule eucaryote.

2. Composition selon la revendication 1, dans laquelle les monomères A identiques ou différents sont choisis parmi les sucres, les esters, les alcools, les acides aminés, les nucléotides, les acides nucléiques ou les protéines.

3. Composition selon la revendication 1 ou 2, dans laquelle le nombre de monomère « a » est tel que la masse desdits polymères de formule (I) est supérieure à 2000 daltons.

4. Composition selon l'une quelconque des revendications 1 à 3, dans laquelle x est compris entre 20 et 150%.

5. Composition selon l'une quelconque des revendications précédentes, dans laquelle le taux de substituions « y » est compris entre 30 et 150%.

6. Composition selon l'une quelconque des revendications précédentes, dans laquelle que ledit polymère biocompatible comprend en outre des groupements chimiques fonctionnels Z, différents de X et Y, capables de conférer audit polymère des propriétés biologiques ou physicochimiques supplémentaires.

7. Composition selon la revendication 7, dans laquelle le taux de substitution de l'ensemble des monomères A par des groupements Z représenté par « z » est compris de 0 à 50%.

8. Composition selon la revendication 7 ou 8, dans laquelle le groupement Z est une substance capable de conférer auxdits polymères une meilleure solubilité ou lipophilie.

9. Composition selon la revendication 8, **caractérisée en ce que** les groupements Z sont identiques ou différents et sont choisis dans le groupe comprenant des acides aminés, des acides gras, des alcools gras, des céramides, ou des dérivés de ceux-ci, ou encore des séquences nucléotidiques d'adressage.

10. Composition selon l'une quelconque des revendications précédentes dans laquelle la cellule eucaryote est choisie dans le groupe comprenant des cellules eucaryotes adultes ou embryonnaires, des cellules de moelle osseuse, des cellules du tissu adipeux.

11. Composition selon l'une quelconque des revendications précédentes dans laquelle ledit biopolymère est administré dans le traitement de lésions tissulaires du système nerveux central dues à une ischémie vasculaire cérébrale par :
- par voie intraveineuse ou par voie intramusculaire a une dose comprise de 0,1 à 5 mg/kg de poids corporel, ou
- par voie orale en 2 à 5 prises égales par jour à hauteur d'un total quotidien de 15 à 500 mg,
- par voie intracrânienne à une dose comprise de 0,001 à 1 mg.ml⁻¹
- par voie sublinguale à jeun d'une solution concentrée aqueuse de 1 à 100mg/ml,
- par voie aérienne par aspersion d'une solution comprenant de 0,1 à 5 mg/kg de poids corporel dudit polymère,
et dans laquelle ladite cellule eucaryote est utilisée dans le traitement par injection dans un délai de 5 minutes à 1 mois après la première administration dudit polymère biocompatible.

12. Kit pharmaceutique pour la prévention et/ou le traitement de lésions tissulaires du système nerveux central dues à une ischémie vasculaire cérébrale comprenant :
i. un polymère biocompatible de formule générale (I) suivante
AaXxYy (I)
dans laquelle :
A représente un monomère,
X représente un groupement R₁COOR₂, ou - R₉(C=O)R₁₀
Y représente un groupement O ou N-sulfonate et répondant à l'une des formules suivante -R₃OSO₃R₄, -R₅NSO₃R₆, -R₇SO₃R₈ dans lesquelles :
R₁, R₃, R₅ et R₉ représentent indépendamment une chaîne hydrocarbonée aliphatique, éventuellement ramifiée et/ou insaturée et éventuellement qui contient un ou plusieurs cycles aromatiques,
R₂, R₄, R₆ et R₈ représentent indépendamment un atome d'hydrogène ou un cation M⁺, et
R₇ et R₁₀ représentent indépendamment une liaison, une chaîne hydrocarbonée aliphatique, éventuellement ramifiée et/ou insaturée, a représente le nombre de monomères,
x représente le taux de substitution des monomères A par des groupements X,
y représente le taux de substitution des monomères A par des groupements Y, et
ii. une cellule eucaryote.

13. Kit pharmaceutique selon la revendication 12 dans lequel le polymère biocompatible est administré
- par voie intraveineuse ou par voie intramusculaire a une dose comprise de 0,1 à 5 mg/kg de poids corporel, ou
- par voie orale en 2 à 5 prises égales par jour à hauteur d'un total quotidien de 15 à 500 mg,
- par voie intracrânienne à une dose comprise de 0,001 à 1 mg.ml-1
- par voie sublinguale à jeun d'une solution concentrée aqueuse de 1 à 100mg/ml,
- par voie aérienne par aspersion d'une solution comprenant de 0,1 à 5 mg/kg de poids corporel dudit polymère,
et dans laquelle ladite cellule eucaryote est utilisable pour injection dans un délai de 5 minutes à 1 mois après la première administration dudit polymère biocompatible.

14. Kit pharmaceutique selon la revendication 12 ou 13 dans lequel le polymère biocompatible et/ou ladite cellule sont administrés sur une période de 1 jours à 3mois

15. Kit pharmaceutique selon l'une quelconque des revendications 12 à 14, dans lequel le polymère biocompatible et/ou ladite cellule dans lequel l'administration est quotidienne, biquotidienne ou hebdomadaire.

16. Utilisation d'une composition pharmaceutique comprenant
- un polymère biocompatible de formule générale (I) suivante
AaXxYy (I)
dans laquelle :
A représente un monomère,
X représente un groupement R₁COOR₂, ou - R₉(C=O)R₁₀
Y représente un groupement O ou N-sulfonate et répondant à l'une des formules suivante -R₃OSO₃R₄, -R₅NSO₃R₆, -R₇SO₃R₈ dans lesquelles :
R₁, R₃, R₅ et R₉ représentent indépendamment une chaîne hydrocarbonée aliphatique, éventuellement ramifiée et/ou insaturée et éventuellement qui contient un ou plusieurs cycles aromatiques,
R₂, R₄, R₆ et R₈ représentent indépendamment un atome d'hydrogène ou un cation M⁺, et
R₇ et R₁₀ représentent indépendamment une liaison, une chaîne hydrocarbonée aliphatique, éventuellement ramifiée et/ou insaturée,
a représente le nombre de monomères,
x représente le taux de substitution des monomères A par des groupements X,
y représente le taux de substitution des monomères A par des groupements Y, et
- une cellule eucaryote.
pour la fabrication d'un médicament pour le traitement de lésions tissulaires du système nerveux central dues à une pathologie hypoxique cérébrale.
